# EUROPÄISCHE PATENTSCHRIFT

(11) **EP 3 988 128 B1**
(45) Veröffentlichungstag und Bekanntmachung des Hinweises auf die Patenterteilung: **12.06.2024**
(21) Anmeldenummer: 21215171.6
(22) Anmeldetag: 14.06.2019
(51) Int. Cl.: A61L 2/08, B65G 25/02

(54) **ANLAGE ZUM STERILISIEREN VON STERILISATIONSEINHEITEN UND VERFAHREN ZUM BETRIEB EINER DERARTIGEN ANLAGE**
SYSTEM FOR STERILISING STERILISATION UNITS AND METHOD FOR OPERATING SUCH A SYSTEM
INSTALLATION PERMETTANT DE STÉRILISER DES UNITÉS DE STÉRILISATION ET PROCÉDÉ PERMETTANT DE FAIRE FONCTIONNER UNE TELLE INSTALLATION

(43) Veröffentlichungstag der Anmeldung: 27.04.2022
(62) Teilanmeldung aus: 19730360.5
(73) Patentinhaber: Framatome GmbH, 91052 Erlangen (DE); BBF Sterilisationsservice GmbH, 71394 Kernen-Rommelshausen (DE)
(72) Erfinder: SIEGELIN, Steffen, 96052 BAMBERG (DE); BIEBER, Oswald, 91336 HEROLDSBACH (DE); JANDL, Johannes, 73630 REMSHALDEN (DE)
(74) Vertreter: Lavoix

(56) Entgegenhaltungen:
- EP-A1- 2 008 668
- US-A- 5 958 336
- US-A1- 2008 299 002

## Beschreibung

Die Erfindung betrifft ferner ein Verfahren zum Betrieb einer Anlage zum Sterilisieren von Sterilisationseinheiten durch Strahlenexposition, insbesondere zum Sterilisieren von medizinischen Objekten enthaltenden Sterilisationseinheiten durch Strahlenexposition. Die Anlage umfasst ein Fördersystem zum Transport der Sterilisationseinheiten durch eine sterilisierende Umgebung längs einer Förderstrecke, wobei die sterilisierende Umgebung radioaktiver Strahlung aus einer Gammastrahlung emittierenden Strahlungsquelle ausgesetzt ist. Zumindest ein Förderabschnitt der Förderstrecke erstreckt sich entlang der Peripherie der Strahlungsquelle. Die Sterilisation der Sterilisationseinheiten erfolgt durch Exposition von Gammastrahlung aus der Strahlungsquelle.

Es ist bekannt, Objekte zur Sterilisation einer hochenergetischen und insbesondere ionisierenden Strahlung, beispielsweise UV-Licht oder radioaktiver Strahlung, auszusetzen. Aus dem Stand der Technik sind zudem Anlagen mit Fördersystemen bekannt, die die zu sterilisierenden Objekte durch einen Bereich oder Raum führen, der dieser Strahlung ausgesetzt ist. Die Sterilisation der Objekte erfolgt somit durch Strahlenexposition.

US 2003/0006378 beschreibt beispielsweise eine Anlage zum Sterilisieren von Objekten, bei denen diese zur Sterilisation mit Hilfe von Bandförderern in die Nähe einer Gammastrahlung emittierenden Strahlungsquelle transportiert werden.

US 5,958,336 beschreibt eine Anlage zum Sterilisieren von Objekten, bei der zum Transport der Objekte beispielsweise ein Hubbalkenförderer zum Einsatz kommt. Die Sterilisation erfolgt durch Bestrahlung mit UV-Licht.

US 2008/299002 A1 beschreibt ein Verfahren zum Sterilisieren durch Strahlung von medizinischen Geräten.

GB 2252316 A offenbart ein System zum Bestrahlung von medizinischen Abfallbehältern.

Es ist Aufgabe der vorliegenden Erfindung, eine Verfahren zum Betrieb einer Anlage zur Sterilisation von Sterilisationseinheiten anzugeben, mit der die Sterilisation effizient und flexibel, insbesondere in Bezug auf die Applikation der geforderten Zieldosen auf die zu sterilisierenden Produkte, durchgeführt werden kann.

Diese Aufgabe wird gelöst durch ein Verfahren zum Betrieb einer Anlage zum Sterilisieren von Sterilisationseinheiten mit den Merkmalen das Patentanspruchs 1.

Vorteilhafte Weiterbildungen der Erfindung sind Gegenstand der abhängigen Ansprüche.

Eine Anlage zum Sterilisieren von Sterilisationseinheiten durch Strahlenexposition, insbesondere zum Sterilisieren von medizinischen Objekten enthaltenden Sterilisationseinheiten durch Strahlenexposition, umfasst ein Fördersystem zum Transport der Sterilisationseinheiten durch eine sterilisierende Umgebung längs einer Förderstrecke. Die sterilisierende Umgebung (auch: heiße Zelle, Bestrahlungsraum, Strahlungsbereich) ist radioaktiver Strahlung aus einer Strahlungsquelle ausgesetzt. Zumindest ein Förderabschnitt der Förderstrecke erstreckt sich entlang der Peripherie der Strahlungsquelle, so dass die Sterilisationseinheiten während des Transports entlang der Förderstrecke der von der Strahlungsquelle emittierten radioaktiven Strahlung ausgesetzt sind. Die Strahlungsquelle emittiert Gammastrahlung und ist beispielsweise als Kobaltquelle, insbesondere als Co 60-Quelle, ausgeführt.

Gemäß der Erfindung umfasst das Fördersystem zumindest einen Hubbalkenförderer mit zumindest einem stationären Tragbalken und zumindest einem beweglichen Hubbalken, der bezüglich des stationären Tragbalkens in einer longitudinalen und einer vertikalen Richtung beweglich ist. Der zumindest eine stationäre Tragbalken weist einen zentralen Bereich zwischen zwei Festlager auf, der über zumindest ein zugbelastetes Zugelement abgestützt ist. Das zumindest eine Zugelement ist am zentralen Bereich und an zumindest einer Vertikalstrebe, die im Bereich zumindest eines der Festlager angeordnet ist, derart befestigt, dass sich das Zugelement in einer Richtung diagonal zu der longitudinalen und der vertikalen Richtung erstreckt.

Die Anlage zeichnet sich insbesondere durch hohe Effizienz und Zuverlässigkeit, durch eine hohe Geschwindigkeit des Fördersystems und durch einen hohen Durchsatz von Bestrahlungsgut aus. Zudem ist eine weitgehend homogene Bestrahlung des Sterilisationsgutes sichergestellt. Die Hubbalken und Tragbalken des zumindest einen Hubbalkenförderers sind vorzugsweise mit minimaler Masse ausgelegt und so positioniert, dass möglichst wenig Abschirmung im relevanten Bestrahlungsbereich entsteht und eine homogene Dosisverteilung auf die entlang der Förderstrecke transportierten Sterilisationseinheiten einwirkt. Damit die Ausführung der Hubbalken und Tragbalken mit möglichst minimaler Masse erfolgen kann, sind die Tragbalken zusätzlich durch die zugbelasteten Zugelemente mechanisch unterstützt. Die diagonale Anordnung der Zugelemente bewirkt, dass die von dem jeweiligen Zugelement verursachte Abschirmung auf unterschiedlichen Höhenniveaus erfolgt. Die Sterilisationseinheiten werden somit im Mittel einem homogenen Bestrahlungsfeld ausgesetzt, wenn diese entlang der Förderstrecke, die durch die der Peripherie der Strahlungsquelle verläuft, bewegt werden.

Die in der Anlage eingesetzten Hubbalkenförderer sind insbesondere derart ausgeführt, dass diese in der unmittelbaren Umgebung einer Strahlungsquelle hoher Intensität mit einer Aktivität von beispielsweise einem bis mehreren Millionen Curie (MCi Bereich) eingesetzt und in Bereichen mit beispielsweise einer Ortsdosisleistung von mehreren Kilogray pro Stunde (kGy/h) und Umgebungstemperaturen von bis zu 70°C dauerhaft und stabil betrieben werden können. Lager, Antriebe, Aktoren, Sensoren oder dergleichen, die im Bestrahlungsfeld eingesetzt werden, bestehen vorzugsweise zumindest zum Teil aus strahlungsresistenten Materialen und umfassen beispielsweise vollmetallische Zylinder ohne organische Dichtungen, Lager ohne Schmiermittel und Sensoren ohne Elektronik und organische Stoffe.

Hubbalkenförderer können in Anlagen zum Sterilisieren von Sterilisationseinheiten besonders vorteilhaft eingesetzt werden, da diese eine Beförderung des Bestrahlungsgut in vorwärts- und Rückwärtsrichtung ermöglichen. Auf diese Weise kann die Verweildauer der Sterilisationseinheiten in der sterilisierenden Umgebung flexibel angepasst werden, um eine definierte Strahlungsexposition zu bewirken.

In Ausgestaltungen ist der zumindest eine Hubbalkenförderer pneumatisch betrieben und umfasst insbesondere zumindest einen Vorschubzylinder, der als Antrieb für die longitudinale Bewegung des beweglichen Hubbalkens ausgebildet ist, und zumindest einen Hubzylinder, der als Antrieb für die vertikale Bewegung des beweglichen Hubbalkens ausgebildet ist. Pneumatische Antriebe sind insbesondere für den zuverlässigen Betrieb in einer Hochdosisumgebung bevorzugt, da diese eine erhöhte Strahlenbeständigkeit aufweisen. Elektrische Antriebe und hydraulische Antriebe sind ungeeignet für den Einsatz in einer derartigen Hochdosisumgebung. Hydraulische Antriebe weisen typischerweise den Nachteil auf, dass deren Hydraulikflüssigkeit, typischerweise Öl, bei intensiver Strahlenexposition verhärten kann. Die Verwendung von pneumatischen Antrieben bzw. Aktoren zum Antrieb der Hubbalkenförderer erhöht somit die Lebensdauer und Zuverlässigkeit der Anlage.

In Ausgestaltungen ist das Zugelement als Stahlseil ausgebildet.

In Ausgestaltungen ist der bewegliche Hubbalken und/oder stationäre Tragbalken als T-Träger, insbesondere als Doppel-T-Träger, ausgeführt. Der Materialanteil und die Form der Trag- und/oder Hubbalken sind so ausgelegt, dass die von den Sterilisationseinheiten verursachte maximale Last dauerhaft bei minimaler Durchbiegung getragen werden kann. Hierbei ist die elastische Verformung und Durchbiegung bei maximaler Last mit Sterilisationseinheit berücksichtigt. Die Hub- und Tragbalken haben in Ausgestaltungen beispielsweise eine freitagende Länge, die zur Überbrückung des Beckens, in welchem die Strahlungsquelle abgesenkt werden kann, ausreichend ist. Der zumindest eine Hub- und/oder Tragbalken weist daher ein Trägerprofil auf, welches so ausgelegt ist, dass sich möglichst wenig Material zwischen Strahlenquelle und Sterilisationsgut befindet. Im möglichen Ausgestaltungen weisen die Hub- und Tragbalken jeweils Doppel-T-Profile mit einer freitragenden Länge zwischen den Lagern von mehr als 4 m. Die Dimensionen der Hub- und Tragbalken sind in möglichen Ausführungsbeispielen so ausgelegt, dass eine maximale Last von 1000kg getragen werden kann und die elastische Verformung des jeweiligen Hub- oder Tragbalkens bei maximaler Last relativ gering, insbesondere kleiner als 20mm, ist. Der geringe Materialeinsatz hat zur Folge, dass eine geringe Abschirmwirkung zwischen Strahlungsquelle und den Sterilisationseinheiten einwirkt. Das von der Strahlungsquelle bereitgestellte Strahlungsfeld wird somit optimal ausgenutzt und die Bestrahlung der Sterilisationseinheiten erfolgt im Wesentlichen homogen.

In Ausgestaltungen ist der zentrale Bereich durch zumindest zwei Zugelemente abgestützt, die an gegenüberliegenden Vertikalstreben, die jeweils im Bereich eines der Festlager angeordnet sind, befestigt sind. Die Unterstützung des zentralen Bereichs erfolgt insbesondere von zumindest zwei Seiten, wobei die Zugelemente jeweils diagonal zur Förderrichtung verlaufen, um unerwünschte Abschirmungseffekte möglichst zu vermeiden.

In Ausgestaltungen ist die Strahlungsquelle bei Inaktivität der Anlage in einem mit Wasser gefüllten Becken gelagert. Eine Hubeinrichtung ist dazu ausgebildet, die Strahlungsquelle aus dem mit Wasser gefüllten Becken anzuheben und in das mit Wasser gefüllten Becken abzusenken.

In Ausgestaltungen weist die Förderstrecke zumindest zwei Förderabschnitte auf, die in horizontalen Ebenen verlaufen, welche in vertikaler Richtung zueinander beabstandet sind. Diese Ausgestaltungen dienen zur besseren Ausnutzung des von der Strahlungsquelle bereitgestellten Strahlungsfelds. Zudem ist ein Transport der Sterilisationseinheiten in mehreren horizontalen Ebenen vorteilhaft, da hierdurch die Möglichkeit von Mehrfachumläufen um die Strahlungsquelle für individuell ausgewählte Sterilisationseinheiten geschaffen wird, d. h. einzeln ausgewählte Sterilisationseinheiten können bei Bedarf auf einer dazu vorgesehenen horizontalen Ebene mehrmals um die Strahlungsquelle herumgeführt werden, um eine höhere Energiedosis zu deponieren, wobei der kontinuierliche Analgenbetrieb nicht unterbrochen werden muss. Insbesondere ist es möglich, verschiedene Dosisenergien im kontinuierlichen Anlagenbetrieb zu applizieren. Durch die Anordnung der Förderstrecke in mehreren (beliebig vielen) horizontalen Ebenen wird eine Teilbeladung des Fördersystems ermöglicht und es ist kein komplettes Leerfahren der Anlage für die Bestrahlung von Sterilisationseinheiten mit unterschiedlicher Dosis notwendig.

In Ausgestaltungen weist die Förderstrecke zumindest zwei in vertikaler Richtung übereinander angeordnete und parallel zueinander ausgerichtete Förderabschnitte auf. Die übereinander angeordneten Förderabschnitte erstrecken sich innerhalb der sterilisierenden Umgebung entlang der Peripherie der Strahlungsquelle, um den innerhalb der sterilisierenden Umgebung zur Verfügung stehende Raum optimal zu nutzen.

In Ausgestaltungen sind die zumindest zwei übereinander angeordneten Förderabschnitte über zumindest einen Aufzug, der dazu ausgebildet ist, die zu fördernden Sterilisationseinheiten in vertikaler Richtung zu transportieren, verbunden. Vertikale Aufzüge sind hinsichtlich der besseren Ausnutzung des Raums, insbesondere mit Hinblick auf das von der Strahlungsquelle bereitgestellte Strahlungsfeld, vorteilhaft. Zudem dienen die Aufzüge zur Realisierung von gegebenenfalls mehreren Umläufen der Sterilisationseinheiten bzw. des Bestrahlungsgutes innerhalb der heißen Zelle, insbesondere in Abhängigkeit der gewünschten Zieldosis. Es ist in Ausgestaltungen insbesondere vorgesehen, individuell ausgewählte und sensorisch identifizierte Sterilisationseinheiten mit Hilfe des Aufzugs an den Anfang eines Streckenabschnitts der Förderstrecke zurückzubefördern, der durch die sterilisierende Umgebung der Strahlungsquelle verläuft.

In Ausgestaltungen ist der zumindest eine Aufzug als pneumatischer Aufzug ausgebildet. Wie bereits erwähnt, sind pneumatische Antriebe bzw. Aktoren insbesondere für den zuverlässigen Betrieb in einer Hochdosisumgebung bevorzugt, da diese insbesondere gegenüber elektrischen oder hydraulischen antrieben bzw. Aktoren eine erhöhte Strahlenbeständigkeit aufweisen.

In Ausgestaltungen sind die zumindest zwei in vertikaler Richtung übereinander angeordneten und parallel zueinander ausgerichteten Förderabschnitte als Hubbalkenförderer mit jeweils stationären Tragbalken und beweglichen Hubbalken realisiert bzw. ausgebildet. Eine Hubbewegung der übereinander angeordneten, beweglichen Hubbalken in vertikaler Richtung wird mit Hilfe zumindest eines gemeinsamen Hubzylinders angetrieben. Auf diese Weise wird eine kompakte Bauweise vorgeschlagen, bei der die Hubbewegung insbesondere aller übereinander angeordneten Hubbalken mit Hilfe der selben Hubzylinder betrieben wird.

In Ausgestaltungen weist die Förderstrecke zumindest zwei in einer horizontalen Ebene angeordnete und parallel zueinander verlaufende Förderabschnitte auf, wobei insbesondere die Strahlungsquelle zwischen den zumindest zwei parallel zueinander verlaufenden Förderabschnitten angeordnet ist. Vorzugsweise ist die Förderstrecke derart ausgelegt, dass zum Transport von zu bestrahlenden Sterilisationseinheiten vorgesehene Förderabschnitte sich beidseitig von der Strahlungsquelle erstrecken bzw. die Strahlungsquelle zumindest zum Teil von Förderabschnitten der Förderstrecke umfänglich umgeben ist. Auf diese Weise wird das von der Strahlungsquelle bestrahlte Gebiet besser ausgenutzt.

In Ausgestaltungen weist die Förderstrecke zumindest einen Förderabschnitt auf, der einen Rollenförderer umfasst.

In Ausgestaltungen weist die Förderstrecke zumindest einen Förderabschnitt auf, der einen Drehteller umfasst.

In Ausgestaltungen ist der Drehteller als Aufzug ausgebildet oder auf dem Aufzug angeordnet.

In Ausgestaltungen ist ein pneumatisch angetriebener Querschieber dazu ausgebildet, die Sterilisationseinheiten durch Translationsbewegung über einen Abschnitt der Förderstrecke zu fördern. Wie bereits erwähnt, sind pneumatische Antriebe bzw. Aktoren insbesondere für den zuverlässigen Betrieb in einer Hochdosisumgebung bevorzugt, da diese insbesondere gegenüber elektrischen oder hydraulischen Antrieben bzw. Aktoren eine erhöhte Strahlenbeständigkeit aufweisen. Es hat sich gezeigt, dass ein Drehen der Sterilisationseinheiten insbesondere innerhalb der sterilisierenden Umgebung unerwünscht ist, da die Sterilisationseinheiten von allen Seiten gleichmäßig bestrahlt werden, wenn die Sterilisationseinheiten in der gleichen Orientierung um die Strahlungsquelle bewegt werden. Dies bevorzugt den Einsatz der Querschieber, die dazu ausgebildet sind, die Sterilisationseinheiten durch Translationsbewegung zu fördern. Die Orientierung der Sterilisationseinheiten im Raum wird bei der Translationsbewegung nicht geändert. Die Querschieber sind zudem eine platzsparende Lösung, um die Sterilisationseinheiten, insbesondere von einem Hubbalkenförderer zum nächsten Hubbalkenförderer, zu transportieren.

In Ausgestaltungen ist am Eingang der Förderstrecke zumindest eine Registriereinrichtung angeordnet, die dazu ausgebildet ist, kodierte Information, die den zu sterilisierenden Sterilisationseinheiten zugeordnet ist, auszulesen. Die Registriereinrichtung registriert beispielsweise die in einem Wareneingang der Anlage eingehenden Sterilisationseinheiten.

In Ausgestaltungen ist die Registriereinrichtung dazu ausgebildet, optisch kodierte Information, die auf den Sterilisationseinheiten aufgebracht ist, auszulesen. Die kodierte Information ist beispielsweise als Barcode oder QR-code optisch kodiert. In anderen Ausgestaltungen sind RFID-Tags, Transponder oder andere elektronisch bzw. elektromagnetisch auslesbare Mittel als Träger der kodierten Information ausgebildet. Entsprechend ist die Registriereinrichtung in solchen Ausgestaltungen als Lesegerät zum Auslesen von elektromagnetischen Felder ausgebildet.

In Ausgestaltungen sind Sensoren entlang der Förderstrecke, insbesondere innerhalb der sterilisierenden Umgebung, angeordnet, die dazu ausgebildet ist, Daten, insbesondere Positionsdaten, zu erfassen, die Information über den Fortgang des Transports der jeweiligen Sterilisationseinheit beinhalten.

In Ausgestaltungen steht die Registriereinrichtung und/oder die Sensoren mit einer Steuereinrichtung in Verbindung. Die Steuereinrichtung ist dazu ausgebildet, in Abhängigkeit der ausgelesenen kodierten Information und/oder der sensorisch erfassten Daten, insbesondere in Abhängigkeit der Positionsdaten und/oder in Abhängigkeit der zu applizierende Dosisenergie, zumindest einen Prozessparameter, insbesondere Fördergeschwindigkeit (Taktzeit), Bestrahlungsdauer und/oder Anzahl der Umläufe in der sterilisierenden Umgebung, der Anlage anzupassen. Die Steuereinrichtung berechnet insbesondere die Verweildauer der Sterilisationseinheit innerhalb des sterilisierenden Bereiches in Abhängigkeit der zugeordneten Zieldosis.

Gemäß der Erfindung erfolgt die Sterilisation der Sterilisationseinheiten durch Exposition von Gammastrahlung. Die Sterilisationseinheiten werden zumindest abschnittsweise entlang der Förderstrecke mittels zumindest einen Hubbalkenförderer transportiert, der zumindest einen stationären Tragbalken und zumindest einen beweglichen Hubbalken, der bezüglich des stationären Tragbalkens in einer longitudinalen und einer vertikalen Richtung beweglich ist, umfasst. Die Sterilisationseinheiten werden individuell unterschiedlichen Dosisenergien im kontinuierlichen Anlagenbetrieb ausgesetzt, wobei der kontinuierliche Bestrahlungszyklus nicht unterbrochen wird.

Das vorgeschlagene Verfahren realisiert das Sterilisieren der Sterilisationseinheiten mit Hilfe einer Strahlenquelle derart, dass individuelle Zieldosen auf einzelne Sterilisationseinheiten mit ein- und demselben Fördersystem appliziert werden können, ohne dabei den Bestrahlungszyklus zu unterbrechen. Damit ist ein kontinuierlicher Anlagenbetrieb sichergestellt. Die Anlage muss nicht leergefahren werden, um unterschiedliche Zieldosen zu applizieren, da die Hubbalkenförderer insbesondere hinsichtlich ihrer Taktfrequenz und Fördergeschwindigkeit, insbesondere von der Steuereinrichtung, individuell ansteuerbar sind. Eine Ansteuerung der Hubbalkenförderer zum Fördern der Sterilisationseinheiten über zumindest einen Abschnitt der Förderstrecke kann insbesondere derart erfolgen, dass der Transport in oder entgegen einer Förderrichtung erfolgt.

In Ausgestaltungen ist jeder Sterilisationseinheit kodierte Information, insbesondere optisch kodierte Information, individuell zugeordnet. Die kodierte Information wird zumindest am Eingang des Fördersystems ausgelesen und charakterisiert zumindest eine zu applizierende Dosisenergie bzw. Zieldosis. Die kodierte Information kann insbesondere in Form von Barcodes oder QR-Codes optisch kodiert vorliegen. Die derartig kodierten Sterilisationseinheiten werden in Ausgestaltungen am Wareneingang, beispielsweise mit Hilfe einer Registriereinrichtung, insbesondere mit Hilfe eines Barcode- oder einer QR-Code-Scanners eingelesen. Die kodierte Information legt fest, welche Zieldosis bzw. zu applizierende Dosisenergie den einzelnen Sterilisationseinheiten zugeordnet ist. Zusätzlich kann die kodierte Information die Identität der jeweiligen Sterilisationseinheit eindeutig festlegen, so dass insbesondere die der Weg der Sterilisationseinheiten im Bestrahlungsbereich individuell überwacht und nachvollzogen werden kann.

In Ausgestaltungen ermittelt eine Steuereinrichtung einen Prozessparameter, insbesondere Fördergeschwindigkeit, Bestrahlungsdauer, Länge der Förderstecke in der sterilisierenden Umgebung und/oder Anzahl der Umläufe in der sterilisierenden Umgebung, der Anlage in Abhängigkeit der erfassten bzw. ausgelesenen kodierten Information. Die Steuerung bzw. Steuereinrichtung ermittelt in Ausgestaltungen beispielsweise, wie viele Umläufe die Sterilisationseinheit im Bestrahlungsraum verbringen muss, um eine vorgegebene Zieldosis zu erreichen. In einer möglichen Ausgestaltung entspricht die Energiedosis pro Umlauf mehrere Kilogray, beispielsweise in etwa 6,25kGy. In Ausgestaltungen wird die pro Umlauf applizierte Energiedosis vorab mittels einer Referenzfahrt bestimmt. Hierzu kann insbesondere eine Referenzfahrt mit einer Sterilisationseinheit erfolgen, die integrierte Dosimeter aufweist. Die in der Referenzfahrt erfassten Daten dienen zur Grundlage der Steuerung und werden beispielsweise in einem Speichermedium der Steuereinrichtung abgelegt. Die Steuereinrichtung ermittelt insbesondere die notwendige Taktzeit, also die Bestrahlungszeit der jeweiligen Sterilisationseinheit auf einer Bestrahlungsposition. Beispielsweise wird ermittelt, wie viele der Umläufe um die Strahlungsquelle benötigt werden, um die vorgegebene Zieldosis zu applizieren. Dabei berechnet die Steuerung bzw. Steuerungseinrichtung insbesondere den Abbrand der Strahlungsquelle, der vorzugsweise für den aktuellen Bestrahlungszyklus, insbesondere kontinuierlich, berücksichtigt wird. Vorzugsweise überwacht eine fehlersichere Steuerung die Taktzeit in den einzelnen Bestrahlungspositionen und die Anzahl der Umläufe um die Strahlungsquelle für jede Sterilisationseinheit. Die Bestrahlungszeit (effektive Aufenthaltsdauer im Strahlenumfeld) wird vorzugsweise kontinuierlich von der fehlersicheren Steuerung überwacht. Im Falle einer erkannten Störung wird das Fördersystem vorzugsweise automatisch gestoppt, die Strahlungsquelle automatisch in das Becken abgesenkt und eine Fehlermeldung optisch oder akustisch über geeignete Ausgabemittel ausgegeben.

In Ausgestaltungen wird die Lage der Sterilisationseinheiten entlang der Förderstrecke, insbesondere innerhalb der sterilisierenden Umgebung, sensorisch erfasst. Die sensorische Erfassung der individuellen Sterilisationseinheiten erfolgt insbesondere zur Überwachung des Fördersystems im Rahmen der vorstehend beschriebenen fehlersicheren Steuerung. Alternativ oder zusätzlich erfolgt die sensorische Erfassung der Sterilisationseinheiten, um einen Prozessparameter anzupassen. So kann beispielsweise eine Sterilisationseinheit, der eine höhere Zieldosis zugeordnet ist, hinsichtlich ihrer Lage erfasst werden und in Abhängigkeit der bereits applizierten Dosisenergie automatisch zum Durchlaufen zusätzlicher Umläufe um die Strahlungsquelle oder in Richtung eines Warenausgangs des Fördersystems weitertransportiert werden.

Die Erfindung wird nachstehend auch hinsichtlich weiterer Merkmale und Vorteile anhand der Beschreibung von Ausführungsbeispielen und unter Bezugnahme auf die beiliegende Zeichnung näher erläutert. Dabei zeigen:
- FIG 1: eine Anlage zur Sterilisation von Sterilisationseinheiten gemäß einem möglichen Ausführungsbeispiel in einer perspektivischen Darstellung;
- FIG 2: der Grundriss einer möglichen Raumaufteilung für eine Anlage gemäß einem Ausführungsbeispiel;
- FIG 3: der Verlauf einer Förderstrecke der Anlage zur Sterilisation von Sterilisationseinheiten gemäß einem Ausführungsbeispiel in einer oberen horizontalen Ebene in einer schematischen Draufsicht;
- FIG 4: der Verlauf einer Förderstrecke der Anlage zur Sterilisation von Sterilisationseinheiten gemäß einem Ausführungsbeispiel in einer unteren horizontalen Ebene in einer schematischen Draufsicht;
- FIG 5: einen Abschnitt eines Fördersystems für die Anlage zur Sterilisation von Sterilisationseinheiten mit Aufzug und Drehteller gemäß einem Ausführungsbeispiel in einer perspektivischen Darstellung;
- FIG 6: einen Abschnitt eines Fördersystems für die Anlage zur Sterilisation von Sterilisationseinheiten mit Querschieber gemäß einem Ausführungsbeispiel in einer perspektivischen Darstellung;
- FIG 7A: eine perspektivische Darstellung eines Hubbalkenförderers für das das Fördersystem gemäß einem möglichen Ausführungsbeispiel in einer ersten Position;
- FIG 7B: eine perspektivische Darstellung eines Hubbalkenförderers für das das Fördersystem gemäß einem möglichen Ausführungsbeispiel in einer zweiten Position;
- FIG 7C: eine perspektivische Darstellung eines Hubbalkenförderers für das das Fördersystem gemäß einem möglichen Ausführungsbeispiel in einer dritten Position;
- FIG 7D: eine perspektivische Darstellung eines Hubbalkenförderers für das das Fördersystem gemäß einem möglichen Ausführungsbeispiel in einer vierten Position;
- FIG. 8: ein vergrößerter Ausschnitt der FIG 1;
- FIG. 9: einen Wareneingang der Anlage zur Sterilisation von Sterilisationseinheiten gemäß einem Ausführungsbeispiel in einer schematischen Darstellung;

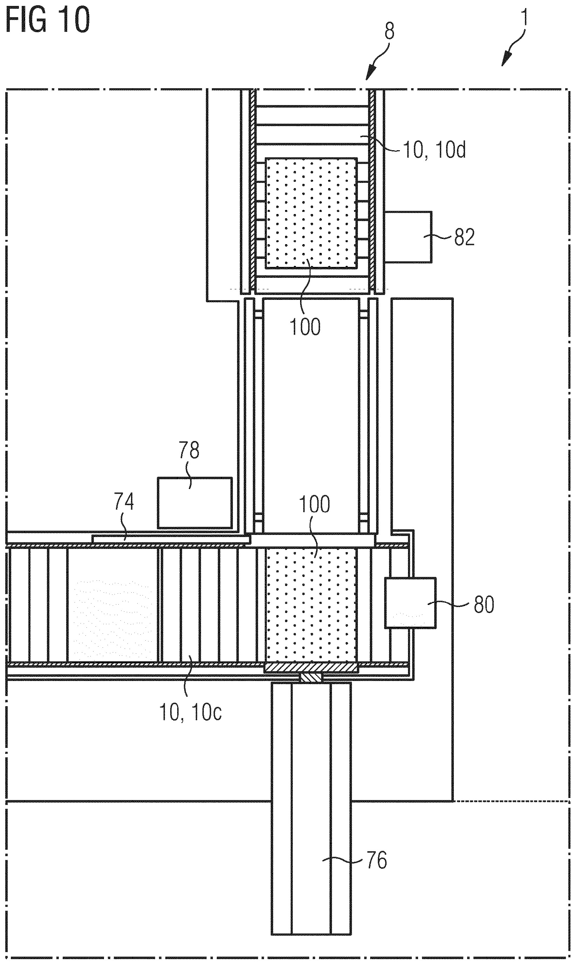

- FIG. 10: einen Warenausgang der Anlage zur Sterilisation von Sterilisationseinheiten gemäß einem Ausführungsbeispiel in einer schematischen Darstellung;

Gleiche oder einander entsprechende Teile sind in allen Figuren mit den gleichen Bezugszeichen versehen.

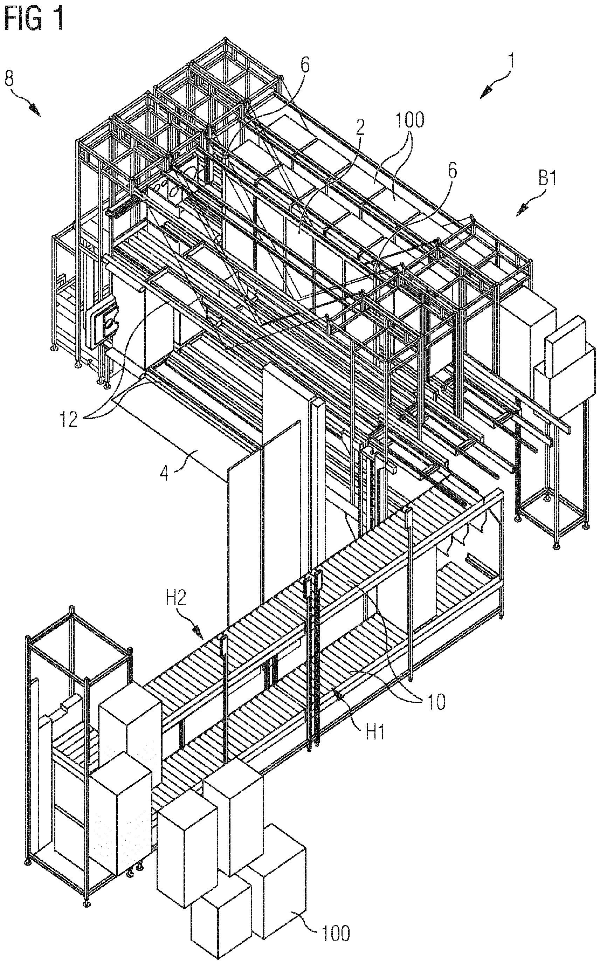

FIG 1 zeigt beispielhaft eine Anlage 1 zur Sterilisation von Sterilisationseinheiten 100 (auch: Bestrahlungsgebinde) durch Strahlenexposition. Die Anlage 1 ist in einem Gebäude untergebracht. Die Bestrahlung der Sterilisationseinheiten 100 erfolgt in einem abgeschirmten Raum, der als "heiße Zelle" B1 bezeichnet wird. In der heißen Zelle B1 befindet sich eine Strahlungsquelle 2, beispielsweise eine Kobaltquelle (Co60-Quelle), die Gammastrahlung emittiert. Die Strahlungsquelle 2 ist nassgelagert, d. h. bei Inaktivität der Anlage 1 ist die Strahlungsquelle 2 in einem Wasser enthaltenden Becken 4 abgesenkt. Zum Absenken der Strahlungsquelle 2 in das Becken 4 bzw. zum Anheben der Strahlungsquelle 2 aus dem Becken 4 ist eine pneumatisch betriebene Hubeinrichtung 6 vorgesehen.

Die Sterilisierung der Sterilisationseinheiten 100 erfolgt durch Bestrahlung mit Gammastrahlung aus der Strahlungsquelle 2. Hierzu ist insbesondere im Raum B1 ein Fördersystem 8 vorgesehen, das die Sterilisationseinheiten 100 entlang einer Förderstrecke fördert, die innerhalb der Peripherie der Strahlungsquelle 2 liegt.

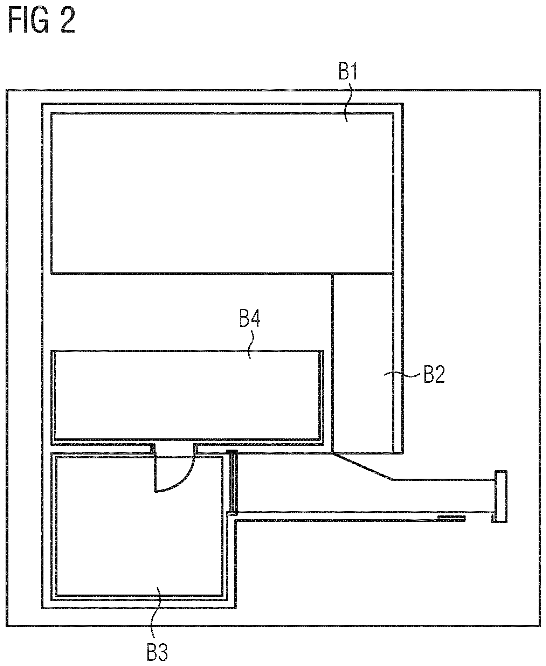

Die Anlage 1 umfasst insbesondere die heiße Zelle B1, das Fördersystem 8, die Strahlungsquelle 2 und typischerweise zusätzlich, wie insbesondere schematisch in Figur 2 dargestellt, einen Maschinenraum B4, einen Steuerungsraum B3 bzw. eine Steuerung der Anlage 1 und Messsysteme.

Die Strahlungsquelle 2 des gezeigten Ausführungsbeispiels umfasst mehrere Kobaltstäbe, die im Betriebsfall in einem Gitter zwischen zwei Abschirmungen in der Mitte des Fördersystems 8 platziert sind. Außerhalb des Sterilisationsbetriebes befindet sich die Strahlungsquelle 2 in dem Becken 4 unterhalb des Fördersystems 8, in das die Strahlungsquelle 2 mit Hilfe der Hubeinrichtung 6 abgesenkt bzw. aus diesem angehoben werden kann. Der Beckenbereich unterhalb der Strahlungsquelle 2 ist stets geöffnet. Der restliche Teil des Beckens 4 ist abgedeckt, um einen bestmöglichen Personenschutz bei abgesenkter Strahlungsquelle 2 sicherzustellen.

Das Fördersystem 8 umfasst Rollenförderer 10 und Hubbalkenförderer 12 (auch: "Walking Beam"), die jeweils Förderabschnitte einer Förderstrecke bilden, entlang der die Sterilisationseinheiten 100 bei Betrieb der Anlage 1 transportiert werden. Wie insbesondere in Fig.1 gezeigt, verläuft die Förderstrecke innerhalb der heißen Zelle B1 mäanderförmig um die Strahlungsquelle 2 herum. Die Förderstrecke ist im exemplarisch gezeigten Beispiel in zwei horizontalen Ebenen H1, H2 angeordnet, die voneinander in vertikaler Richtung beabstandet sind. In anderen Ausführungen kann die Förderstrecke insbesondere auf mehr als zwei zueinander in vertikaler Richtung beabstandeter Ebenen verlaufen. Zum Fördern von Sterilisationseinheiten 100 zwischen den verschiedenen horizontalen Ebenen H1, H2 sind Aufzüge 16 vorgesehen, die dazu ausgebildet sind, die Sterilisationseinheiten 100 entsprechen in vertikaler Richtung zu befördern.

Innerhalb der heißen Zelle B1 erfolgt die Förderung der Sterilisationseinheiten 100 mittels Hubbalkenförderer 12. Außerhalb der heißen Zelle B1, insbesondere im Bereich B2 (siehe insbesondere Fig. 2), der einem Bestrahlungsraum mit geringer Ortsdosisleistung entspricht, sind zum Befördern der zu sterilisierenden bzw. sterilisierten Objekte Rollenförderer 10 angeordnet.

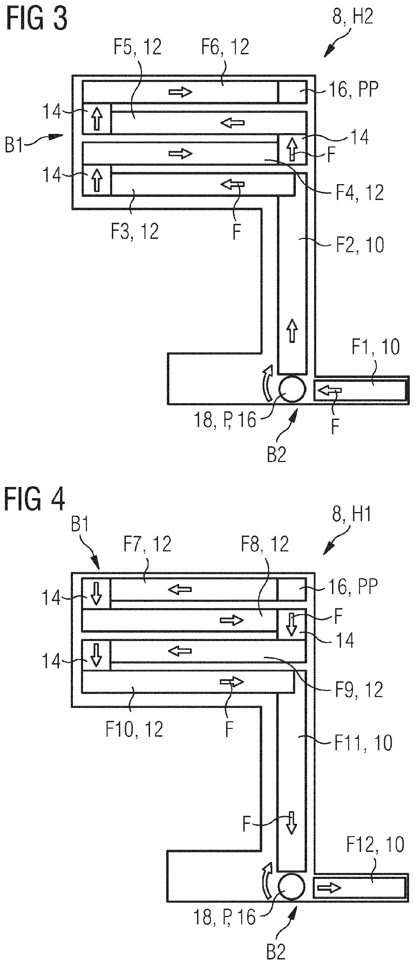

Fig. 3 und 4 zeigen den Verlauf der Förderstrecke in einer schematischen Draufsicht. Fig. 3 zeigt dabei den Verlauf der Förderstrecke in der oberen horizontalen Ebene H2. Entsprechend zeigt Fig. 4 den Verlauf der Förderstrecke in der unteren horizontalen Ebene H1.

Die obere horizontale Ebene H2 ist beispielsweise für den Transport der Sterilisationseinheiten 100 in die heiße Zelle B1 hinein ausgelegt. Entsprechend kann die untere horizontale Ebene H1 für den Transport der Sterilisationseinheiten 100 aus der heißen Zelle B1 hinaus ausgelegt sein. Dabei kann jedoch das Fördersystem 8 insbesondere dazu ausgelegt sein, den Transport in entsprechend entgegengesetzter Förderrichtung zu realisieren. Die Förderrichtung F ist in Fig. 3 und Fig. 4 schematisch durch Pfeile illustriert.

Die Förderstrecke des Fördersystems 8 in der oberen horizontalen Ebene H2 umfasst Förderabschnitte F1 bis F6. Die Förderstrecke des Fördersystems 8 in der unteren horizontalen Ebene H1 umfasst Förderabschnitte F7 bis F12. Die Förderabschnitte F1 und F12 bzw. F2 und F11 bzw. F3 und F10 bzw. F4 und F9 bzw. F5 und F8 bzw. F6 und F7 liegen jeweils übereinander und verlaufen parallel zueinander. Die Förderabschnitte F1, F2, F11 und F12 verlaufen durch den Bereich B2 (vgl. insbesondere Fig. 2) und sind als Rollenförderer 10 ausgebildet. Die Förderabschnitte F3 bis F10 verlaufen mäanderförmig durch die heiße Zelle B1 (vgl. insbesondere Figuren 1 und 2) und sind als Hubbalkenförderer 12 ausgebildet. Jeweils endseitig am Förderabschnitt F3, F4, F5, F7, F8, F9 ist ein Querschieber 14 angeordnet, der dazu ausgebildet ist, die Sterilisationseinheiten 100 durch Translationsbewegung auf einen in Förderrichtung F nachfolgenden Förderabschnitt F4, F5, F6, F8, F9, F10 zu fördern, ohne dabei die Orientierung der Sterilisationseinheiten 100 innerhalb der heißen Zelle B1 zu verändern. Dies ist vorteilhaft, da dadurch eine gleichmäßige Bestrahlung der Sterilisationseinheiten 100 von allen Seiten sichergestellt ist.

In der beispielhaft in Figuren 3 und 4 illustrierten Ausführung ist ein Querschieber 14 vorgesehen, der dazu ausgebildet ist, Sterilisationseinheiten 100 vom Förderabschnitt F3 auf den Förderabschnitt F4 zu fördern. Weitere Querschieber 14 sind dazu ausgebildet, Sterilisationseinheiten 100 vom Förderabschnitt F4 auf den Förderabschnitt F5 bzw. vom Förderabschnitt F5 auf den Förderabschnitt F6 bzw. vom Förderabschnitt F7 auf den Förderabschnitt F8 bzw. vom Förderabschnitt F8 auf den Förderabschnitt F9 bzw. vom Förderabschnitt F9 auf den Förderabschnitt F10 zu fördern.

Die der unteren und der oberen horizontalen Ebene H1, H2 sind durch Aufzüge 16 verbunden, die eine Förderung der Sterilisationseinheiten in vertikaler Richtung ermöglichen. Auf dem Aufzug 16 an der eingangsseitigen Position P sind Drehteller 18 angeordnet, die eine Rotation der Sterilisationseinheiten 100 im Bereich B2, also außerhalb des Bestrahlungsfeldes der heißen Zelle B1, ermöglicht. Der Aufzug 16 an der Position P ermöglich zudem einen Mehrfachumlauf um die Strahlungsquelle 2, insbesondere entsprechend einer der zu sterilisierenden Sterilisationseinheit 100 individuell zugeordneten Zieldosis bzw. Dosisenergie.

Innerhalb der heißen Zelle B1 ist die Förderstrecke durch Hubbalkenförderer 12 gebildet, so dass die Sterilisationseinheiten 100 gegebenenfalls auch entgegen der Förderrichtung F gefördert werden können, insbesondere um eine Bestrahlung entsprechend einer vorgegebenen Dosisenergie bzw. Zieldosis sicherzustellen.

Der endseitig an der Position PP angeordnete Aufzug 16 verbindet die Förderabschnitte F1 bis F6 der unteren horizontalen Ebene H1 mit den Förderabschnitten F7 bis F12 der oberen horizontalen Ebene H2.

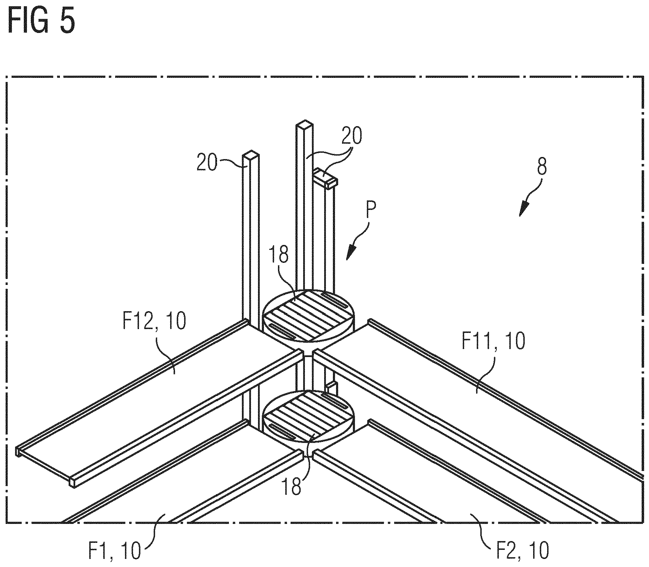

Im Aufzug 16 an Position P sind zur Realisierung des Mehrfachumlaufs zwei Drehteller 18 vorgesehen, die im Detail in der perspektivischen Darstellung der Figur 5 gezeigt sind. Die Drehteller 18 sind in vertikaler Richtung längst der Schienen 20 beweglich geführt. Die Drehteller 18 sind jeweils mit einem Drehantrieb und einem Vorschubantrieb 22 versehen, die insbesondere zwei Aufgaben beim Förderbetrieb in der Anlage 1 erfüllen. Zum einen sind die Drehteller 18 dazu ausgelegt, Sterilisationseinheiten 100 von den durch den Rollförderer 10 gebildeten Förderabschnitt F1 bzw. F11 auf den Förderabschnitt F2 bzw. F12, der ebenfalls durch einen Rollenförderer 10 gebildet ist, zu bewegen. Dabei erfolgt eine motorisch unterstützte 90° Grad Rotation des jeweiligen Drehtellers 19. Zum Transport der Sterilisationseinheiten 100 vom jeweiligen Drehteller 18 auf den jeweiligen Rollenförderer 10 dient der Vorschubantriebs 22, der ebenfalls ein nach Art eines motorisch unterstützten Rollenförderers ausgebildet ist.

Des Weiteren dienen die Drehteller 18 dazu, Sterilisationseinheiten 100, die in einem Durchlauf durch die heiße Zelle B1 noch nicht mit der vorgegebenen Dosisenergie bzw. Zieldosis bestrahlt wurden, wieder an den Anfang der Förderstrecke durch die heiße Zelle B1 zu befördern. Dies erfolgt in dem gezeigten Ausführungsbeispiel dadurch, dass die entsprechenden Sterilisationseinheiten 100 mittels des Aufzugs 18 an der Position P von der unteren horizontalen Ebene H1 zurück auf die obere horizontalen Ebene H2 gefördert werden. Dabei befördert der Drehteller 18 die entsprechende Sterilisationseinheit 100 durch eine Hubbewegung in vertikaler Richtung nach oben. Während dieses Vorgangs wird die Neuaufnahme von weiteren Sterilisationseinheiten 100 vorzugsweise angehalten, um eine unzulässige Bestückung der Rollförderer 10 zu vermeiden.

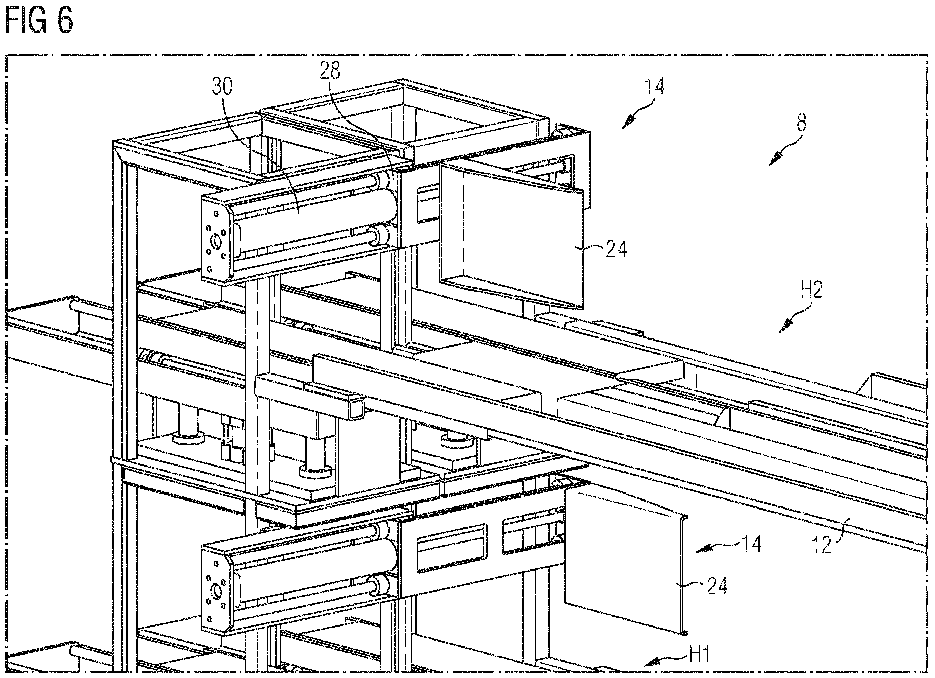

Fig. 6 zeigt Details der Querschieber 14 in einer perspektivischen Darstellung. Zum Ausüben einer Kraft auf die zu transportierenden Sterilisationseinheiten 100 weisen die Querschieber 14 Stempel 24 auf, die in einer Führung 28 linearbeweglich geführt sind und durch Schubzylinder 30 pneumatisch angetrieben sind.

Die pneumatisch betriebenen Stempel 24 schieben im Betrieb die Sterilisationseinheiten 100 mit ihrer Flachseite auf den in Förderrichtung F nächsten Förderabschnitt F4, F5, F6, F8, F9, F10, der als Hubbalkenförderer 12 ausgebildet ist. Dabei wird pro Taktschritt eine Sterilisationseinheit 100 verschoben und der Stempel 24 wieder in Ausgangslage gebracht. Die Stempel 24 in der unteren horizontalen Ebene H1 sind bezüglich der Stempel 24 in der oberen horizontalen Ebene H2 in entgegengesetzter Richtung angeordnet.

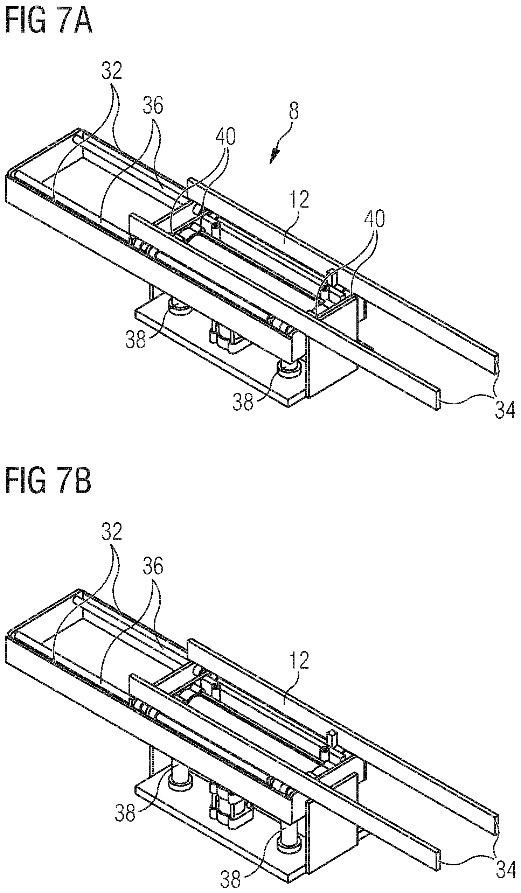

Figuren 7A bis 7D illustriert die Funktionsweise des Hubbalkenförderers 12 in perspektivischen Darstellungen.

Der Hubbalkenförderer 12 gemäß dem dargestellten Ausführungsbeispiel umfasst zwei parallel zueinander angeordnete, stationäre Tragbalken 32 und zwei parallel zueinander angeordnete Hubbalken 34. Die Hubbalken 34 sind pneumatisch angetrieben und relativ zu den Tragbalken 32 sowohl in vertikaler Richtung als auch in longitudinaler Richtung, also in einer Richtung längs der Längsausdehnung der Tragbalken, beweglich geführt. Als Antrieb für die Bewegung der Hubbalken 34 in der longitudinalen Richtung sind pneumatische Vorschubzylinder 36 vorgesehen. Entsprechend sind als Antrieb für die Bewegung der Hubbalken 34 in der vertikalen Richtung sind pneumatische Hubzylinder 38 vorgesehen. In Ausführungsbeispielen, bei denen mehrere Hubbalkenförderer 12 in vertikaler Richtung übereinanderliegend angeordnet sind, ist in vorteilhafter Weise vorgesehen, die übereinanderliegenden Hubbalken 34 aneinander zu koppeln. Auf diese Weise ist ermöglicht, die Hubbewegung der übereinanderliegenden Hubbalken 34 mit den gleichen Hubzylindern 38 anzutreiben. Es ist somit nicht zwingend notwendig, separate Hubzylinder 38 für jeden der übereinaderliegenden Hubbalkenförderer 38 vorzusehen. Dies gilt insbesondere für eine beliebige Anzahl von übereinander angeordneten Förderbahnen mit Hubbalkenförderer 12.

Die Funktion des Hubbalkenförderers 12 ist es, die Sterilisationseinheiten 100 insbesondere von den Rollförderern 10 und den Querschiebern 14 auf und über die Tragbalken 32 zu transportieren. Dies erfolgt in vier Schritten, die entsprechend in Figuren 7A bis 7D illustriert sind.

Ausgangslage ist die in Fig. 7A dargestellte Position. In einem ersten Schritt (in Figuren 7A bis 7D nicht dargestellten) erfolgt eine Bewegung der Hubbalken 34 in vertikaler Richtung, wobei die beweglichen Hubbalken 34 relativ zu oberen Auflagekante der Tragleisten 32 um einige Zentimeter, beispielsweise um etwa 3 cm, nach oben gefahren werden. Dabei werden die auf den Tragbalken 32 ruhenden Sterilisationseinheit 100 angehoben. Die resultierende Positionierung insbesondere der Hubbalken 34 relativ zu den Tragbalken 32 ist in Fig. 7B dargestellt.

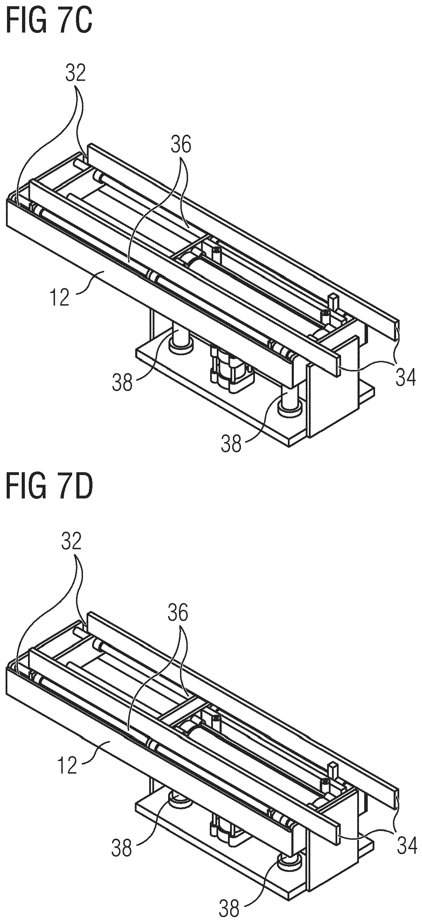

In einem zweiten Schritt erfolgt der Förderhub in Richtung der Förderrichtung F. Hier fahren die Vorschubzylinder 36 entsprechend der Förderlänge pro Taktschritt bevorzugt um mehrere 10 cm, beispielsweise um etwa 60 cm horizontal in longitudinaler Richtung aus, um die Hubbalken 34 entsprechend um diese Förderlänge in Förderrichtung F zu bewegen. Die resultierende Positionierung insbesondere der Hubbalken 34 relativ zu den Tragbalken 32 ist in Fig. 7C dargestellt.

In einem dritten Schritt werden die Sterilisierungseinheiten 100 wieder durch Absenken der Hubzylinder 38 und entsprechend der Hubbalken 34, auf den fest stehenden Tragbalken 32 abgelegt. Die beweglichen Hubbalken 34 werden hierbei unter die feststehenden Tragbalken 32 abgesenkt, beispielsweise derart, dass sich die obere Auflagekante der Hubbalken 34 einige wenige Millimeter, insbesondere etwa 5 mm, unterhalb der oberen Auflagekante der Tragleisten 32 befinden. Die resultierende Positionierung insbesondere der Hubbalken 34 relativ zu den Tragbalken 32 ist in Fig. 7D dargestellt.

In einem vierten Schritt 4 werden die Hubbalken 34 durch die Vorschubzylinder 36 in die Ausgangsposition (Fig. 7A) zurückbewegt.

Die Ein- und/oder Ausfahrgeschwindigkeit der Vorschubzylinder 36 und/oder der Hubzylinder 38 - und damit die Fördergeschwindigkeit entlang der Hubbalkenförderer 12 - kann durch die Regelung der Druckluft eingestellt werden.

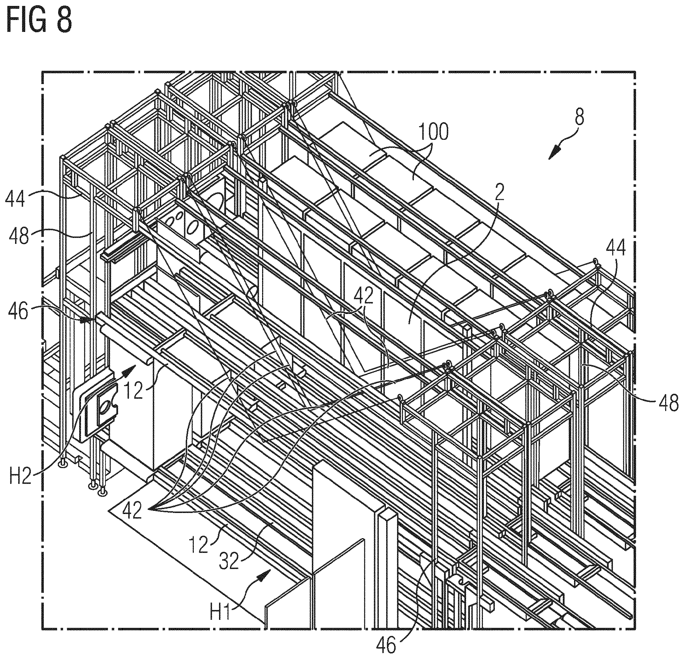

Die Hubbalken 34 sind auf Auflagern 40 und durch außerhalb des relevanten Strahlungsfeldes der Strahlungsquelle 2 angeordnete Gegenlager gelagert. Die Hubbalken 34 und die Tragbalken 32 sind als T-Träger bzw. Doppel-T-Träger ausgebildet. Zur weiteren Erhöhung der mechanischen Stabilität sind die Tragbalken 32 in einem zentralen Bereich durch zugbelastete Zugelemente 42 unterstützt. Dies ist in der Ausschnittvergrößerung der Fig. 8 dargestellt.

Fig. 8 zeigt einen vergrößerten Ausschnitt der Fig. 1. Die Tragbalken 32 sind jeweils endseitig fest an einem Gestell 44 befestigt, das somit ein Festlager 46 für die jeweiligen Tragbalken 32 bildet. Das Gestell 44 umfasst ferner Vertikalstreben 48, an denen die Zugelemente 42, die in dem Ausführungsbeispiel durch Stahlseile realisiert sind, befestigt sind. Die Zugelemente 42 sind zwischen den zentralen Bereichen der jeweiligen Tragbalken 34, die sich in etwa mittig zwischen den von dem Gestell 44 gebildeten Festlagern 46 befinden, und dem oberen Ende des Gestells 44 gespannt. Das Zugelemente 42 erstreckt sich somit in einer Richtung diagonal zur longitudinalen und der vertikalen Richtung. Damit wird erreicht, dass sich die Zugelemente 42 nicht auf dem gleichen Höheniveau befinden, um nachteilige Abschirmungseffekte bei der Sterilisation der Sterilisationseinheiten 100 zu vermeiden, wenn diese entlang der Förderstrecke um die Strahlungsquelle 2 gefördert werden.

Den einzelnen Sterilisationseinheiten 100 ist kodierte Information zugeordnet, die den individuellen Sterilisationsprozess spezifizieren. Zudem enthält die kodierte Information insbesondere individuelle Identifikationsinformation, so dass der Förderprozess der jeweiligen Sterilisationseinheit 100 überwacht werden kann. Die Kodierung erfolgt beispielsweise optisch, insbesondere mit Hilfe eines Etiketts, das mit einem Barcode oder einem QR-Code versehen ist und auf der Sterilisationseinheit 100 angebracht ist. Die kodierte Information enthält insbesondere eine Identifikationsinformation, beispielsweise in Form einer Registriernummer und/oder einer Auftrags-ID, ein Bestrahlungsrezept, das beispielsweise eine zu applizierende Zieldosis, Dosisenergie, Dosis pro Umlauf und/oder Packungsdichte definiert, Information über die notwendige Anzahl von Umläufen um die Strahlungsquelle 2 und/oder Information über den Gebindetyp der jeweiligen Sterilisationseinheit 100.

Die kodierte Information bzw. das Bestrahlungsrezept legt insbesondere Prozessparameter für die nachfolgende Bestrahlung der Sterilisationseinheiten 100 fest. In Abhängigkeit einer zum Zeitpunkt der Bestrahlung vorherrschenden Quellenaktivität kann unter Berücksichtigung einer zu applizierenden Zieldosis (geforderte Zieldosis im Dosisminimum) und der maximalen Dichte des Bestrahlungsgutes (Sterilisationseinheit 100) die Anzahl der Umläufe um die Strahlungsquelle 2 und/oder die Taktzeit ermittelt werden.

In einer möglichen Ausgestaltung der Anlage 1 liegt die maximale Dichte des Bestrahlungsgutes bei etwa 0,25 g/cm³ und die kleinstmögliche Zieldosis, die bei einer beispielhaften Quellenaktivität von 2 MCi und einer Dichte des Bestrahlungsgutes von 0,25 g/cm³ im Dosisminimum bei einem Einfachumlauf um die Strahlenquelle 2 erreicht werden muss, bei etwa ≤ 6,25 kGy. Hieraus ergibt sich beispielsweise in einer möglichen Ausgestaltung der Anlage 1 eine maximale Umlaufzahl von vier Quellenumläufen bei einer Zieldosis von ≤ 25 kGy im Dosisminimum.

Bei der Definition der Prozessparameter wird die Taktzeit wahlweise vom Nutzer vorgegeben oder für den jeweiligen Anwendungszeitpunkt von einer Steuerung automatisch berechnet. Im Rahmen eines Bestrahlungsvorganges, also im Rahmen eines Durchlaufes einer Sterilisationseinheit 100 durch die heiße Zelle B1, wird beispielsweise die Anzahl der Umläufe der Sterilisationseinheit 100 um die Strahlungsquelle 2 in Abhängigkeit einer zugeordneten Zieldosis individuell bestimmt. Ausgehend von der Dichte einer Bestrahlungseinheit von 0,25 g/cm3, einer Quellenaktivität von 2 MCi und eines Vierfachumlaufes der Sterilisationseinheit 100 um die Strahlenquelle 2 ist das Fördersystem 8 einer möglichen Ausgestaltung beispielsweise auf eine Fördergeschwindigkeit ausgelegt, welche die Applikation einer Zieldosis von ≤ 25 kGy im Dosisminimum nach Abschluss des Bestrahlungsvorganges, d. h. beim Verlassen der Heißen Zelle am Zellenausgang, sicherstellt.

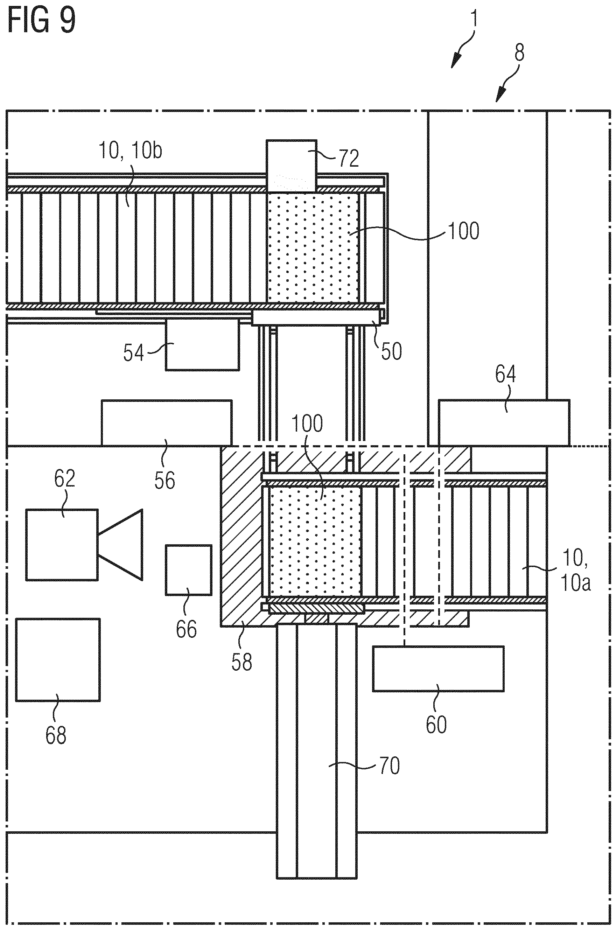

FIG. 9 illustriert den Wareneingang des Fördersystems 8 gemäß einer möglichen Ausführung der Erfindung.

Das Einbringen von Sterilisationseinheiten 100 in die heiße Zelle B1 über den Wareneingang ist über ein pneumatisch angetriebenes Schott 50 mit Endlagensensoren 54 und einem pneumatischen Schieber 70, der einen Schubzylinder aufweist, realisiert. Das Schott 50 wird typischerweise nur zum Einbringen von Sterilisationseinheiten 100 in den Bestrahlungsraum bzw. in die heiße Zelle B1 geöffnet. Ein Sensor 56 erfasst den Eingangsbereich vor dem Schott 50, um zu erkennen, ob die Eingangsöffnung frei ist, und das Schott 50 geschlossen werden kann.

Der direkte Zugang für Personen über den Wareneingang ist in der Regel durch eine Einhausung 58 und dem Schott 50 unterbunden. Eingangsseitig ist ferner ein optischer Sensor 60 und eine Registriereinrichtung 62 angeordnet. Das Einbringen einer Sterilisationseinheit 100 von dem eingangsseitigen Rollenförderer 10a in die Einhausung 58 wird durch einen optischen Sensor 60 und der Registriereinrichtung 62, die dazu ausgebildet, die den einzelnen Sterilisationseinheiten 100 zugeordnete kodierte Information auszulesen, erfasst. Die Registriereinrichtung 62 ist beispielsweise als Barcode oder QR-Code Scanner ausgebildet. Ein zusätzlicher optischer Sensor 64 ist dazu ausgebildet, den Typ der Sterilisationseinheit 100 zu erkennen. Beispielsweise erfasst der optische Sensor 64 die Vertikalausdehnung der am Eingang eingehenden Sterilisationseinheit 100, um zu erkennen, ob beispielsweise ein einzelner Standardkarton oder anderer Typ von Bestrahlungsgebinde eingangsseitig vorliegt. Ein weiterer Sensor 66 ist dazu ausgebildet, zu erkennen, ob sich eine Sterilisationseinheit 100 auf der Eingangsposition innerhalb der Einhausung 58 befindet und zum Transport in den Bestrahlungsraum bzw. in die heiße Zelle B1 bereitsteht.

Dir Registriereinrichtung 62 am Eingang erfasst den bzw. die Barcodes und/oder QR-Codes auf den Sterilisationseinheiten 100 und ermittelt hieraus den zugeordneten Bestrahlungsbindetyp. Aus dem Bestrahlungsbindetyp in Verbindung mit der mittels des optischen Sensors 64 erfassten Höheninformation bestimmt eine Steuerungseinrichtung 68 in einem beispielhaften Anwendungsfall, den konkreten Typ die Sterilisationseinheit 100 am Wareneingang.

Das Einführen der Bestrahlungsgebinde bzw. Sterilisationseinheiten 100 in den Bestrahlungsraum bzw. in die heiße Zelle B1 erfolgt mittels eines pneumatischen Schiebers 70. Die gesteuerte Koordination des pneumatischen Schiebers 70 mit den übrigen in Fig. 9 exemplarisch gezeigten Komponenten der Fördersystems 8 zum Nachfördern von Sterilisationseinheiten 100 geschieht vorzugsweise unter Zuhilfenahme von potentialfreie Kontakten.

Das Erreichen des Übergabepunktes zum Bestrahlungsraum wird mittels des Sensors 66 detektiert. Ist der Übergabepunkt erreicht, so wird von dem in der Steuereinrichtung 68 implementierten, betrieblichen Steuerungssystem folgende Aktionen, insbesondere der Reihe nach, durchgeführt:
- das Schott 50 zum Bestrahlungsraum bzw. zur heißen Zelle B1 wird geöffnet, sofern Sensor 72 meldet, dass die Position hinter dem Schott 50 nicht besetzt ist;
- bei geöffnetem Schott 50 wird die Sterilisationseinheit 100 mittels des pneumatischen Schiebers 70 durch das Schott 50 von dem äußeren Rollenförderer 10a auf den inneren Rollenförderer 10b geschoben;
- Sensor 72 registriert, ob sich eine Sterilisationseinheit 100 im Bereich hinter dem Schott 50 befindet, woraufhin der pneumatische Schieber 70 zurückgezogen wird;
- das Schott 50 wird geschlossen;
- die Endstellungen des Schotts 50 wird sensorisch überwacht;

Die Einhausung 58 dient insbesondere dazu, um bei geöffnetem Schott 50 den Zugang für Personen zum Bestrahlungsraum zu verhindern. Der Eingang zur Einhausung 58 wird mit Hilfe des optischen Sensors 60 und der Registriereinrichtung 62 überwacht. Erkennt der Sensor 60 die Gegenwart eines Objektes und erkennt der Sensor 66 geleichzeitig, dass die Position innerhalb der Einhausung 58 frei ist, so muss als weitere Voraussetzung die Registriereinrichtung 62 einen gültigen Barcode oder QR-Code einlesen, damit der automatische Förderprozess in Gang gesetzt werden kann. Das Schott 50 wird nur bei gültigem QR-Code für die Dauer des Durchschiebens einer Sterilisationseinheit 100 geöffnet. Die Dauer der Öffnung des Schotts 50 wird zeitlich überwacht. Die Sensoren 54, 56, 60, 64, 66 sind Teil einer eingangsseitigen Überwachung der Anlage 1. Sollte ein sicherheitsrelevantes Ereignis erkannt werden, so erfolgt beispielsweise die Ausgabe einer entsprechenden Störmeldung über eine Benutzerschnittstelle, insbesondere eine Anzeigeeinrichtung, ein Monitor, ein Computer oder dergleichen und/oder ein betriebliches Stillsetzen der Anlage 1 mit Absenken der Strahlungsquelle 2 mittels automatischer Ansteuerung der Hubeinrichtung 6. Vorzugsweise wird das geschlossene Schott 50 für den Fall, dass der Sensor 62 kein Hindernis erkennt, automatisch verriegelt. Bei einer Blockierung kann beispielsweise eine automatische Ansteuerung des pneumatischen Schiebers 70 und/oder des Schotts 50 erfolgen, um beispielsweise den Eingangsbereich frei zu räumen.

Am Ausgang der Förderstrecke aus dem Bestrahlungsraum bzw. aus der heißen Zelle B1 ist ein weiteres, pneumatisch betriebenes Schott 74, ein weiterer pneumatischer Schieber 76 mit Schubzylinder und weitere zur Erfassung von Endlagen ausgebildete Sensoren 78 angeordnet. Das Schott 74 wird typischerweise nur zum Ausbringen von Sterilisationseinheiten 100 aus dem Bestrahlungsraum geöffnet. In Ausgestaltung ist insbesondere zusätzlich eine weitere Registriereinrichtung zum Auslesen der den Sterilisationseinheiten 100 zugeordneten Information am Ausgang des Fördersystems 8 angeordnet.

Das Ausbringen von Sterilisationseinheiten 100 aus der Anlage 1 erfolgt typischerweise wie folgt: Die bestrahlten und sterilisierten Sterilisationseinheiten 100 werden von Rollenförderern 10, 10c in der unteren Ebene H1 (vgl. auch Fig. 5) an das Ende der Förderstrecke der heißen Zelle B1 bzw. des Bestrahlungsbereichs transportiert, an dem ein weiterer Sensor 80 angeordnet ist. Der Sensor 80 ist entsprechend dazu ausgebildet, die Gegenwart von Objekten im endseitigen Bereich des Rollenförderers 10c zu detektieren. Wird dort eine Sterilisationseinheit 100 detektiert, so wird mit Hilfe der in der Steuerungseinrichtung 68 implementierten Steuerung das Schott 74 automatisch geöffnet, sofern sich kein Gegenstand im eingangsseitigen Bereich des äußeren Rollenförderers 10d befindet. Diese Position wird durch einen weiteren Sensor 82 überwacht. Bei geöffnetem Schott 74 wird der pneumatische Schieber 76 automatisch aktiviert und die auszubringende Sterilisationseinheit 100 auf den äußeren Rollenförderer 10d. befördert. Der Sensor 82 registriert diesen Transportvorgang. Nach Ansprechen des Sensors 82 wird der pneumatische Schieber 76 automatisch zurückgezogen. Anschließend wird das Schott 74 geschlossen. Für den Fall, dass sich im Bereich des Sensors 82 ein Objekt befinden sollte, kann der vorstehend beschriebene Taktzyklus der Anlage 1 nicht durchgeführt werden. Für diesen Störfall kann insbesondere vorgesehen sein, das Fördersystem 8 automatisch anzuhalten und die Strahlungsquelle 2 mit Hilfe der Hubeinrichtung 6 in das Becken 4 automatisch abzusenken.

Um bei geöffnetem Schott 74 den Zugang für Personen zum Bestrahlungsbereich zu verhindern, wird das Schott 74 in der Regel nur zum Ausbringen einer einzelnen Sterilisationseinheit 100 geöffnet. Anschließend wird das Schott 74 wieder automatisch geschlossen. Die Dauer der Öffnung des Schotts 74 wird zeitlich überwacht. Die Sensoren 78, 60, 82 sind insbesondere Teil einer automatischen Überwachung. Spricht zumindest eine Komponente dieser Überwachung an, so erfolgt beispielsweise die Ausgabe einer entsprechenden Störmeldung über eine Benutzerschnittstelle, insbesondere eine Anzeigeeinrichtung, ein Monitor, ein Computer oder dergleichen. Alternativ oder zusätzlich erfolgt beispielsweise ein betriebliches Stillsetzen der Anlage 1 mit Absenken der Strahlungsquelle 2 in das Becken 4 mittels automatischer Ansteuerung der Hubeinrichtung 6.

Die in Fig. 9 lediglich schematisch angedeutete Steuerungseinrichtung 68 umfasst in Ausgestaltung mehrere Komponenten, wie beispielsweise Prozessoren, Controller, Computer, Server, Clients und/oder speicherprogrammierbare Steuerungen (SPS-Einheiten). Die Steuerungstechnik der Anlage 1 umfasst insbesondere mehreren SPS-Einheiten für die betriebliche und/oder die sicherheitstechnische Steuerung. Insbesondere ist in Ausgestaltungen zumindest eine Steuerung zur Überwachung des Fördersystems 8, zum Heben und Senken der Strahlungsquelle 2 und/oder zur Ansteuerung von Hilfssystemen wie z. B. einer Lüftungsanlage vorgesehen.

Ein Datenserver übernimmt beispielsweise die Verwaltung der Parametrierdaten, zeichnet Messdaten auf, dokumentiert den Sterilisationsvorgang und stellt die Daten für die Benutzer, beispielsweise mit Hilfe von üblichen Computern, wie etwa PCs oder Laptops, zur Verfügung. Die Steuerungseinrichtung 68 kann insbesondere mehrere miteinander drahtlos oder drahtgebunden miteinander vernetzte Rechner, insbesondere Computer, Server und/oder Clients, umfassen.

Die in der Steuerungseinrichtung 68 implementierte Steuerungen können beispielsweise unterschiedlichen Aufgaben dienen, wie etwa der Steuerung des Fördersystems 8 unter Berücksichtigung der vorgegebenen Taktzeit, der Durchlaufzeit und der Umlaufanzahlüberwachung, um insbesondere eine möglichst lückenlose Überwachung der Bestrahlung sicherzustellen. Eine andere mögliche Aufgabe kann die Steuerung der Hubeinrichtung 6 für die Strahlungsquelle 2 oder die Ansteuerung und Überwachung von externen Komponenten und Hilfssystemen betreffen. In Ausgestaltungen dient die Steuerung zudem der Verarbeitung und Dokumentation der Daten aus dem Fördersystem 8 oder dem Messsystem. In Ausgestaltungen dient die Steuerung insbesondere der Verarbeitung und Dokumentation der mittels der Sensoren 56, 60, 64, 66, 72, 78, 80, 82 erfassten den Sensordaten. Zumindest eine Steuerung dient insbesondere zur Überwachung des lückenlosen und ordnungsgemäßen Transports vorzugsweise aller Sterilisationseinheiten 100. Mit Hilfe einer Benutzerschnittstelle können in Ausgestaltungen insbesondere Fördervorgänge und der Status der Anlage 1 animiert dargestellt werden.

Die zu sterilisierenden Produkte werden typischerweise in Sterilisationseinheiten 100, etwa in Behältern oder standardisierten Kartonagen, am Anfang des Fördersystems 8 auf den Rollenförderern 10 der oberen horizontalen Ebene H1 bereitgestellt. Die Behälter bzw. Kartonagen, aus welchen die Sterilisationseinheiten 100 gebildet sind, haben vorzugsweise die gleiche Grundfläche, jedoch können sich die einzelnen Sterilisationseinheiten 100 insbesondere aufgrund von unterschiedlichen Kartonagenkombinationen in der Höhe unterscheiden. Um eine möglichst große Auslastung der Anlage 1 zu realisieren, ist es vorteilhaft das Fördersystem 8 im Taktbetrieb zu betreiben. Ein insbesondere durch einen Hubbalkenförderer 12 realisierter Förderabschnitt F3, F4, F5, F6, F7, F8, F9, F10 entspricht beispielsweise der Länge von 6 bis 9 standardisierten Sterilisationseinheiten 100. Auf den Rollförderern 10, im Bereich der Querschieber 14 und den Aufzügen 16 können insbesondere jeweils eine weitere Sterilisationseinheit 100 angeordnet sein. Taktbetrieb des Fördersystems 8 bedeutet hier insbesondere, dass sich pro Taktzeit eine Sterilisationseinheit 100 schrittweise um jeweils eine Position weiterbewegt.

Während des regulären Förderbetriebs des Fördersystems 8 werden die Sterilisationseinheiten 100, wie vorstehend bereits beschrieben, von dem äußeren Bandförderer 10a über eine Art Schleuse an eine innere Förderanlage übergeben. Am Eingang des Bestrahlungsraumes wird beispielsweise der QR- Code der Sterilisationseinheiten 100 eingelesen und somit die benötigte Dosisleistung, das Bestrahlungsrezept und die benötigte Anzahl an Umläufen an die Steuerung der Steuereinrichtung 68 weitergeben. Eine insbesondere fehlersicher ausgebildete Steuerung prüft die übergebenen Daten und überwacht in Ausgestaltungen die Taktzeit, die Umlaufzeit und die Anzahl der Umläufe der individuellen Sterilisationseinheiten 100 innerhalb der heißen Zelle B1 sowie vorzugsweise alle sonstigen sicherheitsrelevanten Funktionen. Derartige Funktionen betreffen insbesondere den Zugang zum Bestrahlungsraum, beispielsweise über den Wareneingang oder den Warenausgang. Eine betriebliche Steuerung übernimmt die Steuerung vorzugsweise aller relevanten Abläufe, insbesondere derer des Fördersystems 8, wie etwa die Steuerung des Fördersystems 8 unter Berücksichtigung der aktiven Bestrahlungsrezepte, der vorgegebenen Taktzeit und der Umlaufanzahl. Dies beinhaltet in Ausgestaltungen die Ansteuerung von Frequenzumrichtern für die Rollenförderer 10 und/oder die Ansteuerung von Frequenzumrichtern für die Aufzüge 16 und/oder die Ansteuerung von Ventilen für die pneumatische Fördertechnik im Bestrahlungsbereich, insbesondere der Hubbalkenförderer 12 und/oder Querschieber 14 und/oder die Berechnung der Verweildauer bzw. der Taktzeit auf Grundlage der ausgelesenen kodierten Information, insbesondere eines Bestrahlungsrezepts. Weitere relevante Steuerabläufe betreffen insbesondere die Ansteuerung der Hubeinrichtung 6 für die Strahlenquelle 2 und/oder die Ansteuerung und Überwachung von externen Komponenten und Hilfssystemen und/oder das Erfassen der Raumtemperatur und/oder die Ansteuerung von Kältemaschinen, insbesondere mit Umschaltung auf eine redundante Kältemaschine und/oder die Ansteuerung von Kühlwasserpumpen mit automatischer Umschaltung, und/oder die Ansteuerung einer Füllstandergänzung und/oder die Erzeugung von Störmeldungen von externen Aggregaten und/oder die Ansteuerung einer Lüftungstechnik und/oder die Überwachung der Leitfähigkeit des Wassers in einem Kaltwasserkreislauf und/oder die Überwachung des Strahlungspegels im Kaltwasserkreislauf.

## Patentansprüche

1. Verfahren zum Betrieb einer Anlage (1) zum Sterilisieren von Sterilisationseinheiten (100) durch Strahlenexposition, insbesondere zum Sterilisieren von medizinischen Objekten enthaltenden Sterilisationseinheiten (100) durch Strahlenexposition, wobei die Anlage (1) ein Fördersystem(8) zum Transport der Sterilisationseinheiten (100) durch eine sterilisierende Umgebung längs einer Förderstrecke umfasst, wobei die sterilisierende Umgebung radioaktiver Strahlung aus einer Strahlungsquelle (2) ausgesetzt ist und zumindest ein Förderabschnitt (F3, ..., F10) der Förderstrecke sich entlang der Peripherie der Strahlungsquelle (2) erstreckt, wobei die Sterilisation der Sterilisationseinheiten (100) durch Exposition von Gammastrahlung aus der Strahlungsquelle (2) erfolgt, wobei die Sterilisationseinheiten (100) zumindest abschnittsweise entlang der Förderstrecke mittels zumindest eines Hubbalkenförderers (12), der zumindest einen stationären Tragbalken (32) und zumindest einen beweglichen Hubbalken (34) umfasst, der bezüglich des stationären Tragbalkens in einer longitudinalen und einer vertikalen Richtung beweglich ist, transportiert werden , wobei die Sterilisationseinheiten (100) individuell unterschiedlichen Dosisenergien im kontinuierlichen Anlagenbetrieb derart ausgesetzt werden, dass dabei der Bestrahlungszyklus nicht unterbrochen wird, **dadurch gekennzeichnet, dass** der mindestens eine Hubbalkenförderer hinsichtlich seiner Taktfrequenz und Fördergeschwindigkeit individuell ansteuerbar ist.

2. Verfahren nach Anspruch 1, **dadurch gekennzeichnet, dass** jeder der Sterilisationseinheiten (100) kodierte Information, insbesondere optisch kodierte Information, individuell zugeordnet ist, die zumindest am Eingang des Fördersystems (8) ausgelesen wird, wobei die kodierte Information zumindest eine zu applizierende Dosisenergie charakterisiert.

3. Verfahren nach Anspruch 2, **dadurch gekennzeichnet, dass** eine Steuereinrichtung (68) einen Prozessparameter, insbesondere Fördergeschwindigkeit, Bestrahlungsdauer, Länge der Förderstecke in der sterilisierenden Umgebung und/oder Anzahl der Umläufe in der sterilisierenden Umgebung, der Anlage (1) in Abhängigkeit der ausgelesenen, kodierten Information ermittelt.

4. Verfahren nach Anspruch 2 oder 3, **dadurch gekennzeichnet, dass** die Lage der Sterilisationseinheiten (100) entlang der Förderstrecke, insbesondere innerhalb der sterilisierenden Umgebung, sensorisch erfasst wird.

## Claims

1. A method for operating a system (1) for sterilizing sterilization units (100) by radiation exposure, in particular, sterilization units (100) containing medical objects by radiation exposure, wherein the system (1) comprises a conveyance system (8) for transporting sterilization units (100) through a sterilizing environment along a conveying path, wherein the sterilizing environment is exposed to radioactive radiation from a radiation source (2) and at least one conveyance section (F3, ..., F10) of the conveying path extends along the periphery of the radiation source (2), wherein the sterilization of the sterilization units (100) takes place by exposure to gamma radiation from the radiation source (2), wherein the sterilization units (100) are transported at least in sections along the conveying path by means of at least one lifting beam conveyor (12), which comprises at least one stationary supporting beam (32) and at least one movable lifting beam (34), which is movable with respect to the stationary supporting beam in a longitudinal and a vertical direction, wherein the sterilization units (100) are exposed individually to different doses of energy in the continuous operation of the system in such a manner that the irradiation cycle is not thereby interrupted, **characterized in that** the at least one lifting beam conveyor can be individually controlled with regard to its clock frequency and conveying speed.

2. Method according to claim 1, **characterised in that** each of the sterilisation units (100) is individually assigned coded information, in particular optically coded information, which is read out at least at the input of the conveyor system (8), whereby the coded information characterises at least one dose energy to be applied.

3. Method according to claim 2, **characterised in that** a control device (68) determines a process parameter, in particular conveyor speed, irradiation duration, length of the conveyor section in the sterilising environment and/or number of cycles in the sterilising environment, of the system (1) as a function of the read-out, coded information.

4. Method according to claim 2 or 3, **characterised in that** the position of the sterilisation units (100) along the conveyor section, in particular within the sterilising environment, is detected by sensors.

## Revendications

1. Procédé de fonctionnement d'une installation (1) pour la stérilisation d'unités de stérilisation (100) par exposition aux rayonnements, en particulier pour la stérilisation d'unités de stérilisation (100) contenant des objets médicaux par exposition aux rayonnements, l'installation (1) comprenant un système de convoyage (8) pour transporter les unités de stérilisation (100) à travers un environnement de stérilisation le long d'une voie de convoyage, l'environnement de stérilisation étant exposé à un rayonnement radioactif provenant d'une source de rayonnement (2) et au moins une section de convoyage (F3, ..., F10) de la voie de convoyage s'étendant le long de la périphérie de la source de rayonnement (2), la stérilisation des unités de stérilisation (100) s'effectuant par exposition au rayonnement gamma provenant de la source de rayonnement (2), les unités de stérilisation (100) étant transportées, au moins par sections, le long de la voie de convoyage au moyen d'au moins un convoyeur à barres de levage (12), qui comprend au moins une barre de support fixe (32) et au moins une barre de levage mobile (34), qui est mobile par rapport à la barre de support fixe dans une direction longitudinale et une direction verticale, les unités de stérilisation (100) étant exposées individuellement à différentes énergies de dose en fonctionnement continu de l'installation, de telle sorte que le cycle d'irradiation, **caractérisé en ce qu'**au moins un convoyeur à barres de levage peut être commandé individuellement en ce qui concerne sa fréquence de cycle et sa vitesse de convoyage, ne soit pas interrompu.

2. Procédé selon la revendication 1, **caractérisé en ce que** des informations codées, en particulier des informations codées optiquement, qui sont lues au moins à l'entrée du système de convoyage (8), sont associées individuellement à chacune des unités de stérilisation (100), les informations codées caractérisant au moins une énergie de dose à appliquer.

3. Procédé selon la revendication 2, **caractérisé en ce qu'**un dispositif de commande (68) détermine un paramètre de processus de l'installation (1), en particulier la vitesse de convoyage, la durée d'irradiation, la longueur de la voie de convoyage dans l'environnement de stérilisation et/ou le nombre de cycles dans l'environnement de stérilisation, en fonction de l'information codée lue.

4. Procédé selon la revendication 2 ou 3, **caractérisé en ce que** la position des unités de stérilisation (100) est détectée par des capteurs le long de la voie de convoyage, en particulier à l'intérieur de l'environnement de stérilisation.
